# EUROPEAN PATENT SPECIFICATION

(11) **EP 1 453 488 B1**
(45) Date of publication and mention of the grant of the patent: **07.10.2009**
(21) Application number: 02756411.1
(22) Date of filing: 09.07.2002
(51) Int. Cl.: A61K 9/28, A61K 9/68, A61K 47/38

(54) **IMPROVED EDIBLE FILM FORMULATIONS CONTAINING MALTODEXTRIN**
VERBESSERTE ESSBARE FILMFORMULIERUNGEN MIT MALTODEXTRIN
FORMULATIONS DE FILM COMESTIBLE AMELIOREES CONTENANT DE LA MALTODEXTRINE

(30) Priority: 30.07.2001 US 682164; 09.01.2002 US 44105
(43) Date of publication of application: 08.09.2004
(73) Proprietor: WM. WRIGLEY JR. COMPANY, Chicago, Illinois 60611 (US)
(72) Inventor: CHAPDELAINE, Albert, H., Naperville, IL 60453 (US); ZYCK, Daniel, North Riverside, IL 60546 (US); DZIJA, Michael, J., LaGrange Park, IL 60526 (US)
(74) Representative: Hayes, Adrian Chetwynd
(86) International application number: PCT/US2002/021591
(87) International publication number: WO 2003/011259

(56) References cited:
- WO-A-00/45794
- WO-A2-02/43657
- US-A- 5 470 581
- US-A- 5 679 389
- DATABASE WPI Week 199342 Derwent Publications Ltd., London, GB; AN 1993-330524 XP002403158 -& JP 05 236885 A (TAZAWA T) 17 September 1993 (1993-09-17)

## Description

### BACKGROUND OF THE INVENTION

The present invention generally relates to edible compositions. More specifically, the present invention relates to edible film formulations.

Of course, oral cleansing and breath freshening are an important part of everyday life. In order to facilitate proper oral hygiene, oral cleansing and breath freshening practices should be conducted repeatedly throughout the day.

However, oral cleansing and breath freshening may be difficult or inconvenient at times, depending on the nature of the breath freshening desired and the situation in which the breath freshening must occur. Brushing, flossing, cleaning your tongue and gargling using a variety of devices and compositions are common oral care practices well-suited for the privacy of one's home. But, such devices and compositions are less convenient to use away from the home where bathroom facilities might be scarce, unavailable or unsanitary.

To deal with this issue, less obtrusive oral products have been developed. These include breath-freshening gums, lozenges, mouth sprays and edible films.

A number of different edible film compositions are currently available for consumption. Many of the products use pullulan, see for instance JP 5-236 885 A. However, pullulan is an expensive ingredient and may have limited availability. Other edible materials have been employed as a substitute for pullulan within edible film compositions. These materials include modified starches and cellulosics. Unfortunately, such materials typically lack one or more of pullulan's desirable film properties. These properties include, for example, rapid dissolution, clean mouth feel, clean flavor and ease of manufacture.

A need, therefore exists, for improved edible film formulations that exhibit the desirable film properties that are exhibited by pullulan-based edible films. WO 02/43657 A, which is prior art under Article 54(3) EPC, discloses a pullulan free edible film composition.

### SUMMARY OF THE INVENTION

The present invention provides improved edible film formulations and methods of making and using same. The edible films include at least three types of film forming agents other than pullulan that are readily available and can be made at lower costs. In this regard, the mixture of film forming agents, namely maltodextrins, hydrocolloids and fillers, can be used to prepare "stand alone" films that can provide oral cleansing and breath freshening effects while displaying clean flavor and mouth feel, rapid dissolution and ease of manufacture. Medicaments and other additive agents can also be incorporated into the edible films thereby providing effective oral treatment, such as oral cleansing and breath freshening.

To this end, in an embodiment of the present invention, a stand alone edible film for oral mucoadhesion is provided. The stand alone edible film includes at least three types of film forming agents and not including pullulan wherein the film forming agents comprise a maltodextrin, a hydrocolloid and a filler, and wherein the maltodextrin comprises 5% to 60% by dry weight of the film.

In another preferred embodiment of the present invention, an edible film is provided including a maltodextrin, a hydrocolloid and a bulk filler and not including a pullulan, wherein the maltodextrin comprises 5% to 60% by dry weight of the film. In a preferred embodiment, the maltodextrin has a dextrose equivalent of up to 20.

In yet another embodiment, a method of producing an edible film for oral muchoadhesion is provided. The method includes the steps of preparing a base solution including at least three types of film forming agents other than pullulan and processing the base solution to form the edible film wherein the film forming agents comprise a maltodextrin, a hydrocolloid and a filler, and wherein the maltodextrin comprises 5% to 60% by dry weight of the film.

In still yet another embodiment the use of a food-grade film including at least three types of film forming agents and not including pullulan, wherein the film forming agents comprise a maltodextrin, a hydrocolloid and a filler, wherein the maltodextrin comprises 5% to 60% by dry weight of the film, and a medicament for the manufacture of a medicament for oral treatment in an oral cavity is provided.

In still yet another embodiment, a food-grade film including at least three types of film forming agents and not including pullulan, wherein the film forming agents comprise a maltodextrin, a hydrocolloid and a filler, wherein the maltodextrin comprises 5% to 60% by dry weight of the film, and a medicament for use in a method of oral treatment in an oral cavity is provided.

It is, therefore, an advantage of the present invention to provide edible films for mucoadhesion that include at least three types of film forming agents other than pullulan.

Another advantage of the present invention is to provide edible films that include a medicament for cleansing an oral cavity and freshening breath upon consumption.

A further advantage of the present invention is to provide a method for delivering the medicament into an oral cavity for treating same.

Moreover, an advantage of the present invention is to provide a stand alone film.

Additional features and advantages of the present invention are described in, and will be apparent in, the detailed description of the presently preferred embodiments.

### DETAILED DESCRIPTION OF THE PRESENTLY PREFERRED EMBODIMENTS

The present invention provides edible film formulations for oral mucoadhesion and methods of using and making same. In particular, the edible films of the present invention include at least three types of film forming agents other than pullulan.

Applicants have uniquely discovered that the use of a mixture of at least three types of film forming agents, such as maltodextrins, fillers (e.g., microcrystalline cellulose (MCC)) and hydrocolloids (e.g., sodium alginate), can be effectively utilized to prepare "stand alone" edible films. The edible films are composed of ingredients that are readily available, can be prepared at lower costs and display similar properties as compared to edible films composed of pullulan. In this regard, the edible films can provide a physiologically acceptable film, which is suitably adapted to adhere to oral surfaces of an oral cavity and rapidly dissolve therein.

The edible films of the present invention can be utilized to deliver or release oral care agent(s). Such agents include, anti-microbial agents and salivary stimulants to treat, for example, halitosis, dental plaque, gingivitis, xerostomia, dry mouth, like oral conditions or combinations thereof. Further, the oral care edible film can act as a breath freshener effective against malodor.

The oral cleansing and breath freshening effects of the edible film of the present invention can be achieved by entrapping the oral care agents within the oral cavity to provide extended efficacy. In this regard, the highly dissolvable edible film can act as a medium through which a pharmaceutically active oral agent can be administered via a mucous membrane of the oral cavity.

In an embodiment, the edible or food-grade film of the present invention includes at least three types of film forming agents other than pullulan. The edible films can also include a medicament for oral cleansing and/or breath freshening. Further, the edible films can include a variety of other suitable ingredients, such as softeners, colorants, flavoring agents, emulsifiers, surfactants, thickening agents, binding agents, sweeteners, fragrances, other like ingredients or combinations thereof.

In an embodiment, the edible films include a mixture of at least three types of film forming agents, such as maltodextrins, fillers and hydrocolloids. It should be appreciated that the edible film of the present invention can be composed of one or more different compounds associated with each of the at least three types of film forming agents.

Maltodextrins belong to a category of chemical products known as "starch hydrolysates." Manufacturers produce starch hydrolysates by putting starch through a hydrolysis, a chemical reaction with water. Hydrolysis breaks down the starch and converts some of the starch to dextrose. With adjustments, this process yields more or less dextrose.

Maltodextrins are therefore classified by dextrose equivalence or DE. Dextrose equivalents are a measure of the reducing sugars present calculated as dextrose and expressed as a percentage of the total dry substance. Maltodextrins can have a dextrose equivalent of up to 20. At above 20 DE, the product is then classified as corn syrup solids, is completely soluble, and therefore imparts significant sweetness.

Maltodextrins having lower DE values generally offer higher viscosity and better film formation while higher DE values yield more sweetness, solubility, plasticity, and hygroscopicity. Further, for the purposes of this invention, lower DE values offer several processing advantages over higher DE values. Lower DE values improves flexibility of the film which reduces cracking and flaking during slitting and cutting. Being less hygroscopic, lower DE maltodextrins pick up less moisture during slitting and cutting, helping to prevent blocking during shelf life.

In an embodiment, the maltodextrin has a DE of less than 20. In another embodiment, the maltodextrin has a DE of less than or equal to 10. In a further embodiment, the maltodextrin has a DE of 1 or less.

In an embodiment, maltodextrin having a DE of less than 20, constitutes between 5% to about 60% by dry weight, or 20% to about 40% by dry weight of the edible film. Maltodextrins can be derived from several different cereal sources, corn (waxy and common), followed by potato, rice and tapioca.

The hydrocolloid can provide thickness and decrease brittleness of the edible films. The hydrocolloid can include any suitable type, amount and number of hydrocolloids. In an embodiment, the hydrocolloid can constitute between about 10% to about 50% by dry weight of the edible film, preferably about 30% to about 40% by dry weight. The hydrocolloid can be derived from, for example, natural seaweeds, natural seed gum, natural plant exudates, natural fiber extracts, biosynthetic gums, gelatins, biosynthetic process starch or cellulosic materials, alginates, sodium alginate, calcium alginate, carrageenans, guar gum, locust gum, tara gum, gum arabic, ghatti gum, agar gum, xanthan gum, pectin, other like hydrocolloid source material or combinations thereof.

Any suitable food-grade bulk filler can also be added to the edible film. This can reduce any "slimy" texture as well as provide structure to the film thereby making it more palatable. In an embodiment, the filler can constitute about 1% to about 30% by dry weight of the film, preferably about 5% to about 15% by dry weight. The filler can include, for example, microcrystalline cellulose, cellulose polymers, such as wood, magnesium and calcium carbonate, ground limestone, silicates, such as magnesium and aluminum silicate, clay, talc, titanium dioxide, mono-calcium phosphate, di-calcium phosphate, tri-calcium phosphate, other like bulk fillers or combinations thereof.

It is believed that the unique mixture of at least three film forming agents other than pullulan, namely, a maltodextrin, a hydrocolloid and a bulk filler, can provide a "stand alone" edible film composition which exhibits many of the same desirable properties exhibited by the more expensive pullulan-based edible film. Applicants have desirably discovered that the pullulan-free edible film formulation of the present invention can exhibit, for example, clean mouth feel, clean favor and ease of manufacture similar to currently available pullulan-bascd films.

As previously discussed, a variety of other suitable ingredients can be added to the edible film of the present invention. For example, any suitable medicament for oral cleansing, breath freshening or the like can be added to the film formulation. The medicaments can include, for example, a pH control agent, such as urea and buffers, inorganic components for tartar or caries control, such as phosphates and fluorides, a breath freshening agent, such as zinc gluconate, an anti-plaque/anti-gingivitis agent, such as chlorohexidene, CPC, and triclosan, a saliva stimulating agent including, for example, food acids such as citric, lactic, maleic, succinic, ascorbic, adipic, fumaric and tartaric acids, a pharmaceutical agent, a nutraceutical agent, a vitamin, a mineral, other like medicaments or combinations thereof.

The medicaments can be delivered or released into the oral cavity for effective oral treatment, such as oral cleansing and/or breath freshening. In this regard, the film forming agents of the edible film can act to entrap the medicaments within the oral cavity thereby providing extended efficacy thereof In doing so, it is believed that the pullulan free edible film compositions of the present invention more uniformly release the medicament into the oral cavity for absorption via open wounds or mucous membrane in a greater manner than could be previously achieved. Moreover, it is also believed that the mixture of film-forming agents of the present invention can entrap the medicament within the oral cavity for an extended period of time to prolong and enhance the effects of the medicament. In addition, by extending the contact time of the medicament within the oral cavity, the medicament is absorbed to a greater extent thereby increasing its bioavailability.

If reduced levels of film forming agents are utilized, softeners can be used to reduce the brittleness of the resulting films. The softeners, which are also known as plasticizers or plasticizing agents, generally constitute between about 0% to about 20% by dry weight of the film, preferably about 2% to about 10% by dry weight. The softeners can include plasticizers containing, for example, sorbitol and other polyols, glycerin, polyethylene glycol, propylene glycol, hydrogenated starch hydrolysates, corn syrups, other like material or combinations thereof.

The edible film formulations of the present invention can also include colorants or coloring agents which can be used in any suitable amount to produce the desired color. Coloring agents can include, for example, natural food colors and dyes suitable for food, drug and cosmetic applications. The colorants are typically known as FD&C dyes and lakes.

A variety of flavoring agents can also be added to the edible films. Any suitable amount and type of artificial and/or natural flavoring agents can be used in any sensorially acceptable fashion. For example, the flavor can constitute about 0.1 % to about 20% by dry weight of the film, preferably about 10% to 15%. The flavoring agent can include, for example, essential oils, synthetic flavors or mixtures including but not limited to oils derived from plants and fruits such as citrus oil, fruit essences, peppermint oil, spearmint oil, other mint oils, clove oils, oil of wintergreen, anise and the like, flavor oils with germ killing properties such as menthol, eucalyptol, eugenol, thymol, like flavoring agents or combinations thereof.

The flavor can be enhanced and evenly distributed throughout the product by emulsification. Any suitable amount and type of natural and/or synthetic food-grade emulsifier can be used. For example, the emulsifier can include lecithin, food-grade non-ionic emulsifiers, such as fatty acids (C₁₀-C₁₈), mono and diacyl glycerides, ox bile extract, polyglycerol esters, polyethylene sorbitan esters, propolyene glycol, sorbitan monopalmitate, sorbitan monosterate, sorbitan tristerate, other like emulsifiers or combinations thereof.

The flavors can be emulsified by any suitable emulsification process, such as mechanical processing, vigorous stirring, intense pressure fluctuations that occur in turbulent flow such as homogenization, sonication, colloid milling and the like.

The present invention provides methods of producing the edible film formulations. In general, the edible film formulations are prepared by forming a base solution that includes at least three types of film forming agents, such as maltodextrins, hydrocolloids and fillers and processing the base solution to form an edible film. Typically, the base solution is prepared by adding an initial mixture of dry ingredients to water that is stirred.

To the base solution, additional ingredients, such as flavor/emulsifier blends, sweeteners, softeners, color, the like or combinations thereof, can be added. In an embodiment, the solution is stirred continuously and heated at a temperature ranging from about 40°C to about 50°C. The solution then can be dried in any suitable manner, thereby, forming the edible film.

It should be appreciated that any suitable type, number and arrangement of process procedures or steps (i.e., mixing, heating, drying, cooling, addition of ingredients), process parameters (i.e., temperature, pressure, pH, process times) or the like can be utilized.

By way of example and not limitation, the following examples illustrate various embodiments of the edible film formulations of the present invention.

### EXAMPLES

**(% dry weight)**

| **Ingredient** | **Example 1** | **Example 2** | **Example 3** | **Example 4** | **Example 5** |
|---|---|---|---|---|---|
| Maltodextrin | 36.00 | 32.45 | 31.15 | 30.00 | 37.00 |
| Sodium Alginate | 22.15 | 20.00 | 19.00 | 31.15 | -- |
| Carageenan | -- | -- | -- | -- | 23.15 |
| Microcrystalline Cellulose | 20.00 | 18.00 | 17.00 | 17.00 | 18.00 |
| Gum Arabic | -- | -- | 11.00 | -- | -- |
| Glycerin | 7.30 | 15.00 | 7.30 | 7.30 | 7.30 |
| Flavor | 11.00 | 11.00 | 11.00 | 11.00 | 11.00 |
| Lecithin | 2.00 | 2.00 | 2.00 | 2.00 | 2.00 |
| High Intensity Sweetener | 1.50 | 1.50 | 1.50 | 1.50 | 1.50 |
| Color | 0.05 | 0.05 | 0.05 | 0.05 | 0.05 |
| | 100.00 | 100.00 | 100.00 | 100.00 | 100.00 |

**(% dry weight)**

| **Ingredient** | **Example 6** | **Example 7** | **Example 8** | **Example 9** | **Example 10** |
|---|---|---|---|---|---|
| Maltodextrin | 25.95 | 50.00 | 35.00 | 43.00 | 26.00 |
| Sodium Alginate | 22.50 | -- | 19.00 | -- | 12.00 |
| Calcium Alginate | -- | 15.15 | -- | 13.25 | -- |
| Carageenan | -- | -- | -- | -- | 12.00 |
| Microcrystalline Cellulose | 25.75 | 9.00 | 20.00 | 13.00 | 25.00 |
| Calcium Carbonate | -- | 2.45 | -- | -- | -- |
| Glycerin | 12.25 | 10.00 | 8.00 | -- | 9.5 |
| Sorbitol | -- | -- | -- | 6.00 | 1.55 |
| Popylene Glycol | -- | -- | 3.65 | 5.00 | -- |
| Menthol | 1.00 | 0.05 | -- | 1.25 | -- |
| Eucalyptol | -- | 0.05 | -- | 1.00 | -- |
| Maleic Acid | -- | -- | -- | -- | 1.35 |
| Citric Acid | -- | -- | , 1.25 | -- | 1.00 |
| Chlorohexidene | 1.85 | -- | -- | 1.00 | -- |
| Triclosan | -- | 1.25 | -- | 1.00 | -- |
| Flavor | 9.40 | 11.00 | 12.00 | 14.00 | 10.00 |
| High Intensity Sweetener | 1.25 | 1.00 | 1.05 | 1.45 | 1.50 |
| Color | 0.05 | 0.05 | 0.05 | 0.05 | 0.10 |
| | 100.00 | 100.00 | 100.00 | 100.00 | 100.00 |

### EXAMPLES

**(% dry weight)**

| **Ingredient** | **Example 11** | **Example 12** | **Example 13** | **Example 14** | **Example 15** |
|---|---|---|---|---|---|
| Star-Dri® 1 Maltodextrin | 30.00 | -- | -- | 29.00 | 22.00 |
| Star-Dri® 10 Maltodextrin | -- | 30.00 | 29.00 | -- | -- |
| Sodium Alginate | 27.08 | 27.08 | 25.30 | 25.30 | 9.00 |
| Carageenan | 5.22 | 5.22 | 9.00 | 9.00 | 10.00 |
| Microcrystalline Cellulose | 10.00 | 10.00 | 7.00 | 7.00 | 3.30 |
| Glycerin | 4.00 | 4.00 | 7.70 | 7.70 | 3.00 |
| Flavor | 8.90 | 8.90 | 9.00 | 9.00 | 10.92 |
| Hydroxylated Lecithin | 1.00 | 1.00 | -- | -- | 2.00 |
| Lecithin | -- | -- | 1.50 | 1.50 | -- |
| High Intensity Sweetener | 1.50 | 1.50 | 1.10 | 1.10 | 2.00 |
| Color | 0.30 | 0.30 | 0.40 | 0.40 | 0.40 |
| Water | 10.00 | 10.00 | 10.00 | 10.00 | 5.00 |
| Total | 100.00 | 100.00 | 100.00 | 100.00 | 100.00 |

| | | | | | |
|---|---|---|---|---|---|
| * Star-Dri® is a product of the Staley Corporation. Both maltodextrins are derived from waxy corn. | | | | | |

**(% dry weight)**

| **Ingredient** | **Example 16** | **Example 17** | **Example 18** | **Example 19** | **Example 20** |
|---|---|---|---|---|---|
| Maltrin® M050 (4-7 DE) | 25.00 | -- | -- | -- | 20.00 |
| N-Zorbit® M | -- | 32.45 | -- | 20.50 | 20.00 |
| Star-Dri® 10 Maltodextrin | -- | -- | 42.95 | 12.50 | -- |
| (10 DE) | | | | | |
| Sodium Alginate | 15.00 | 20.00 | -- | -- | -- |
| Calcium Alginate | 15.00 | -- | 11.00 | -- | 17.00 |
| Carageenan | -- | -- | 12.00 | 15.00 | 19.00 |
| Microcrystalline Cellulose | 23.00 | 20.00 | 11.00 | 18.00 | -- |
| Gum Arabic | -- | -- | -- | 5.00 | -- |
| Calcium Carbonate | -- | -- | 1.50 | -- | 8.00 |
| Glycerin | 8.00 | 12.00 | -- | 10.00 | 4.50 |
| Sorbitol | -- | -- | 1.50 | -- | -- |
| Propylene Glycol | -- | -- | 5.00 | -- | -- |
| Menthol | 1.00 | 0.05 | -- | 2.00 | -- |
| Eucalyptol | -- | 1.45 | -- | 0.60 | 2.50 |
| Maleic Acid | -- | -- | 1.00 | -- | -- |
| Citric Acid | 1.00 | -- | 1.00 | -- | -- |
| Chlorohexidene | -- | 1.50 | -- | 1.00 | -- |
| Triclosan | -- | -- | -- | 1.00 | 1.00 |
| Flavor | 10.00 | 11.00 | 12.00 | 13.00 | 6.00 |
| High Intensity Sweetener | 2.00 | 1.50 | 1.00 | 1.40 | 2.00 |
| Color | -- | 0.05 | 0.05 | 0.05 | -- |
| | 100.00 | 100.00 | 100.00 | 100.00 | 100.00 |

| | | | | | |
|---|---|---|---|---|---|
| * Maltrin® is a product of the Grain Processing Corporation. It is derived from dent com. * N-Zorbit® is a product of National Starch and Chemical. It is derived from tapioca starch. | | | | | |

### PREPARATION OF EXAMPLES 1-5

Examples 1-5 were prepared by making edible films from maltodextrin. A mixture of dry ingredients, such as maltodextrin, sodium alginate and microcrystalline cellulose, was slowly added to water while stirring. The solution was continually stirred and heated to boiling. After which, it was removed from the heat and cooled to a temperature of about 35°C to about 40°C. The other ingredients, such as flavor/emulsifier blends, sweeteners, softeners and color, were added to the solution while stirring and at a temperature of about 35°C to about 40°C. The solution was spread onto a glass plate by utilizing a draw down blade. A film was formed on the glass plate by drying the solution in an oven for about 15 minutes at 40°C. After which, the film was removed from the plate and cut into desired pieces.

The edible films of Examples 1-5 were compared for sensory analysis. All of the films exhibited differences in texture and mouth feel. Example 1 provided good mouth feel, clean flavor, fast dissolution and flexibility. In comparison, Example 2 had good flexibility, but was found to be too sticky to handle. In Example 3, the addition of gum arabic showed no improvement in flexibility. Example 4 was slightly slimy and showed no improvement in flexibility. Example 5 was slimy, slow to dissolve, yet sufficiently flexible.

### PREPARATION OF EXAMPLES 11-15

Examples 11-15 were prepared by making edible films from maltodextrin. A mixture of dry ingredients, such as maltodextrin and sodium alginate, was slowly added to water while stirring. The solution was continually stirred and heated. The other ingredients, such as flavor/emulsifier blends, sweeteners, softeners and color, were added to the maltodextrin/alginate solution while stirring and at a temperature of about 50°C to about 60°C. The solution was spread onto a glass plate by utilizing a draw down blade. A film was formed on the glass plate by drying the solution in an oven for about 15 minutes at 50°C. After which, the film was removed from the plate and cut into desired pieces.

Benchmark sensory analysis of Examples 11-15 demonstrated comparable sensory properties. Analysis also determined that while having similar sensory profiles, the 1 DE maltodextrin film offered several processing advantages over 10 DE maltodextrin film. The 1 DE film improved the flexibility of the film, which reduced cracking and flaking during slitting and cutting. Since lower DE maltodextrins are less hygroscopic, the 1 DE film picked up less moisture during slitting and cutting which helped prevent blocking during shelf life. Overall, the lower DE maltodextrin films provided better film-forming capabilities than maltodextrin films with higher DE's.

## Claims

1. A stand alone edible film for oral mucoadhesion comprising at least three types of film forming agents and not including pullulan, wherein the film forming agents comprise a maltodextrin, a hydrocolloid and a filler, and wherein the maltodextrin comprises 5% to 60% by dry weight of the stand alone edible film.

2. The edible film of claim 1 wherein the filler comprises 1% to 30% by dry weight of the stand alone edible film.

3. The edible film of claim 2 wherein the filler is selected from microcrystalline cellulose, cellulose polymers including wood, magnesium and calcium carbonate, ground limestone, silicates including magnesium and aluminium silicate, clay, talc, titanium dioxide, mono-calcium phosphate, di-calcium phosphate, tri-calcium phosphate and combinations thereof.

4. The edible film of claim 1 wherein the hydrocolloid further comprises 10% to 50% by dry weight of the stand alone edible film.

5. The edible film of claim 4 wherein the hydrocolloid is selected from natural seaweeds, natural seed gums, natural plant exudates, natural fiber extracts, biosynthetic gums, gelatins, biosynthetic processed starch or cellulosic materials, alginates, sodium alginate, calcium alginate, carrageenan, guar gum, locust gum, tara gum, gum arabic, ghatti gum, agar gum, xanthan gum, pectin and combinations thereof.

6. The edible film of claim 1 wherein the stand alone edible film includes a medicament.

7. The edible film of claim 6 wherein the medicament is selected from a pH control agent, an oral care agent, a breath freshening agent, a pharmaceutical agent, a nutraceutical agent, a salivary stimulant agent, a vitamin, a mineral, an anti-microbial agent, an anti-plaque agent, an anti gingivitis agent, a tartar or caries control agent and combinations thereof.

8. The edible film of claim 1 wherein the filler is a bulk filler.

9. The edible film of claims 1 or 8 wherein the maltodextrin has a dextrose equivalent of less than 20.

10. The edible film of claim 8 wherein the maltodextrin constitutes 20% to 40% by dry weight of the edible film.

11. The edible film of claim 8 wherein the hydrocolloid constitutes 30% to 40% by dry weight of the edible film.

12. The edible film of claim 8 wherein the bulk filler comprises 1% to 30% by dry weight of the edible film.

13. The edible film of claim 1 wherein the maltodextrin has a dextrose equivalent of 10 or less.

14. The edible film of claim 1 or claim 8 wherein the maltodextrin has a dextrose equivalent of 1 or less.

15. A method of producing a stand alone edible film for oral mucoadhesion comprising the steps of:
preparing a base solution including at least three types of film forming agents other than pullulan, wherein the film forming agents comprise a maltodextrin, a hydrocolloid and a filler; and
processing the base solution to form the edible film, wherein the maltodextrin comprises from 5% to 60% by dry weight of the edible film.

16. The method of claim 15 wherein the hydrocolloid comprises 10% to 50% by dry weight of the edible film and the filler comprises 1% to 30% by dry weight of the edible film.

17. The method of claim 15 wherein the maltodextrin has a dextrose equivalent of less than 20.

18. The method of claim 15 wherein the base solution is processed by adding a therapeutically effective amount of a medicament selected from a pH control agent, an oral care agent, a breath freshening agent, a pharmaceutical agent, a nutraceutical agent, a salivary stimulant agent, a vitamin, a mineral, an anti-microbial agent, an anti-plaque agent, an anti-gingivitis agent, a tartar or caries control agent and combinations thereof.

19. The edible film according to any of claims 1 to 14 further comprising a medicament for use in a method of oral treatment in an oral cavity.

20. Use of the edible film according to claim 19 in the manufacture of a medicament for oral treatment in an oral cavity.

21. The use of claim 20 wherein the hydrocolloid comprises 10% to 50% by dry weight of the edible film and a filler comprises from 1% to 30% by dry weight of the edible film.

22. The use of claim 20 wherein the maltodextrin has a dextrose equivalent of less than 20.

23. The use of claim 20 wherein the medicament is selected from a pH control agent, an oral care agent, a breath freshening agent, a pharmaceutical agent, a nutraceutical agent, a salivary stimulant agent, a vitamin, a mineral, an anti-microbial agent, an anti-plaque agent, an anti-gingivitis agent, a tartar or caries control agent or combinations thereof.

24. The use of claim 20 wherein the medicament is released in the oral cavity to treat halitosis, dental plaque, gingivitis, xerostomia, dry mouth, oral malodour or combinations thereof.

## Patentansprüche

1. Eigenständiger essbarer Film für die orale Mukoadhäsion, umfassend mindestens drei Typen an filmbildenden Mitteln und nicht umfassend Pullulan, wobei die filmbildenden Mittel ein Maltodextrin, ein Hydrokolloid und einen Füllstoff umfassen und wobei das Maltodextrin 5% bis 60% bezogen auf das Trockengewicht des eigenständigen essbaren Films umfasst.

2. Essbarer Film nach Anspruch 1, wobei der Füllstoff 1 % bis 30% bezogen auf das Trockengewicht des eigenständigen essbaren Films umfasst.

3. Essbarer Film nach Anspruch 2, wobei der Füllstoff ausgewählt ist aus mikrokristalliner Zellulose, Zellulose-Polymeren einschließlich Holz, Magnesium und Calciumcarbonat, gemahlenem Kalkstein, Silicaten einschließlich Magnesium- und Aluminium-Silicat, Lehm, Talk, Titandioxid, Monocalciumphosphat, Dicalciumphosphat, Tricalciumphosphat und Kombinationen davon.

4. Essbarer Film nach Anspruch 1, wobei das Hydrokolloid weiter 10% bis 50% bezogen auf das Trockengewicht des eigenständigen essbaren Films umfasst.

5. Essbarer Film nach Anspruch 4, wobei das Hydrokolloid ausgewählt ist aus natürlichen Seetangen, natürlichen Samengummis, natürlichen Pflanzenexudaten, natürlichen Faserextrakten, biosynthetischen Gummis, Gelatinen, biosynthetisch verarbeiteter Stärke oder Zellulosematerialien, Alginaten, Natriumalginat, Calciumalginat, Carrageenan, Guargummi, Johannesbrotgummi, Taragummi, Gummi Arabikum, Ghattigummi, Agar-Gummi, Xanthan-Gummi, Pektin und Kombinationen davon.

6. Essbarer Film nach Anspruch 1, wobei der eigenständige essbare Film ein Medikament umfasst.

7. Essbarer Film nach Anspruch 6, wobei das Medikament ausgewählt ist aus einem pH-Kontrollmittel, einem Mundpflegemittel, einem Atemerfrischungsmittel, einem pharmazeutischen Mittel, einem nutrazeutischen Mittel, einem Mittel zur Speichelstimulanz, einem Vitamin, einem Mineral, einem antimikrobiellen Mittel, einem Anti-Plaquemittel, einem Anti-Gingivitismittel, einem Zahnstein- oder Karies-Kontrollmittel und Kombinationen davon.

8. Essbarer Film nach Anspruch 1, wobei der Füllstoff ein Massenfüllstoff ist.

9. Essbarer Film nach Anspruch 1 oder 8, wobei das Maltodextrin ein Dextroseäquivalent von weniger als 20 hat.

10. Essbarer Film nach Anspruch 8, wobei das Maltodextrin 20% bis 40% bezogen auf das Trockengewicht des essbaren Films ausmacht.

11. Essbarer Film nach Anspruch 8, wobei das Hydrokolloid 30% bis 40% bezogen auf das Trockengewicht des essbaren Films ausmacht.

12. Essbarer Film nach Anspruch 8, wobei der Massenfüllstoff 1 % bis 30% bezogen auf das Trockengewicht des essbaren Films umfasst.

13. Essbarer Film nach Anspruch 1, wobei das Maltodextrin ein Dextroseäquivalent von 10 oder weniger hat.

14. Essbarer Film nach Anspruch 1 oder Anspruch 8, wobei das Maltodextrin ein Dextroseäquivalent von 1 oder weniger hat.

15. Verfahren zur Herstellung eines eigenständigen essbaren Films für die orale Mukoadhäsion, umfassend die Schritte:
Zubereiten einer Basenlösung einschließlich mindestens drei Typen an filmbildenden Mitteln außer Pullulan, wobei die filmbildenden Mittel ein Maltodextrin, ein Hydrokolloid und einen Füllstoff umfassen; und
Verarbeiten der Basenlösung, um den essbaren Film zu bilden, wobei das Maltodextrin von 5% bis 60% bezogen auf das Trockengewicht des essbaren Films umfasst.

16. Verfahren nach Anspruch 15, wobei das Hydrokolloid 10% bis 50% bezogen auf das Trockengewicht des essbaren Films und der Füllstoff 1 % bis 30% bezogen auf das Trockengewicht des essbaren Films umfasst.

17. Verfahren nach Anspruch 15, wobei das Maltodextrin ein Dextroseäquivalent von weniger als 20 hat.

18. Verfahren nach Anspruch 15, wobei die Basenlösung verarbeitet wird, indem eine therapeutisch wirksame Menge eines Medikaments, ausgewählt aus einem pH-Kontrollmittel, einem Mundpflegemittel, einem Atemerfrischungsmittel, einem pharmazeutischen Mittel, einem nutrazeutischen Mittel, einem Mittel zur Speichelstimulanz, einem Vitamin, einem Mineral, einem antimikrobiellen Mittel, einem Anti-Plaquemittel, einem Anti-Gingivitismittel, einem Zahnstein- oder Karies-Kontrollmittel und Kombinationen davon, zugegeben wird.

19. Essbarer Film nach einem der Ansprüche 1 bis 14, weiter umfassend ein Medikament zur Verwendung in einem Verfahren zur oralen Behandlung in einer Mundhöhle.

20. Verwendung eines essbaren Films nach Anspruch 19 zur Herstellung eines Medikaments zur oralen Behandlung in einer Mundhöhle.

21. Verwendung nach Anspruch 20, wobei das Hydrokolloid 10% bis 50% bezogen auf das Trockengewicht des essbaren Films und ein Füllstoff von 1% bis 30% bezogen auf das Trockengewicht des essbaren Films umfasst.

22. Verwendung nach Anspruch 20, wobei das Maltodextrin ein Dextroseäquivalent von weniger als 20 hat.

23. Verwendung nach Anspruch 20, wobei das Medikament ausgewählt ist aus einem pH-Kontrollmittel, einem Mundpflegemittel, einem Atemerfrischungsmittel, einem pharmazeutischen Mittel, einem nutrazeutischen Mittel, einem Mittel zur Speichelstimulanz, einem Vitamin, einem Mineral, einem antimikrobiellen Mittel, einem Anti-Plaquemittel, einem Anti-Gingivitismittel, einem Zahnstein- oder Karies-Kontrollmittel oder Kombinationen davon.

24. Verwendung nach Anspruch 20, wobei das Medikament in der Mundhöhle freigesetzt wird, um Halitose, Plaque, Gingivitis, Xerostomie, trockenen Mund, Mundgeruch oder Kombinationen davon zu behandeln.

## Revendications

1. Film comestible isolé pour muco-adhérence buccale, comprenant au moins trois types d'agents filmogènes et ne comprenant pas de pullulane, dans lequel les agents filmogènes comprennent une maltodextrine, un hydrocolloïde et une charge, et dans lequel la maltodextrine représente 5 % à 60 % en poids sec du film comestible isolé.

2. Film comestible suivant la revendication 1, dans lequel la charge représente 1 % à 30 % en poids sec du film comestible isolé.

3. Film comestible suivant la revendication 2, dans lequel la charge est choisie entre la cellulose microcristalline, des polymères cellulosiques y compris le bois, le carbonate de magnésium et de calcium, le calcaire broyé, des silicates comprenant le silicate de magnésium et d'aluminium, une argile, le talc, le dioxyde de titane, le phosphate monocalcique, le phosphate dicalcique, le phosphate tricalcique et leurs associations.

4. Film comestible suivant la revendication 1, dans lequel l'hydrocolloïde représente en outre 10 % à 50 % en poids sec du film comestible isolé.

5. Film comestible suivant la revendication 4, dans lequel l'hydrocolloïde est choisi entre des hydrocolloïdes naturels d'algues, des gommes naturels de graines, des exsudats naturels de végétaux, des extraits naturels de fibres, des gommes biosynthétiques, des gélatines, un amidon traité biosynthétique ou des matières cellulosiques, des alginates, l'alginate de sodium, l'alginate de calcium, la carraghénine, la gomme guar, la gomme de caroube, la gomme tara, la gomme arabique, la gomme ghatti, la gomme d'agar-agar, la gomme xanthane, la pectine et leurs associations.

6. Film comestible suivant la revendication 1, ledit film isolé comprenant un médicament.

7. Film comestible suivant la revendication 6, dans lequel le médicament est choisi entre un agent d'ajustement du pH, un agent pour les soins buccaux, un agent rafraîchisseur d'haleine, un agent pharmaceutique, un agent nutraceutique, un agent stimulant la production de salive, une vitamine, une substance minérale, un agent antimicrobien, un agent anti-plaque, un agent anti-gingivite, un agent destiné à lutter contre le tartre et les caries et leurs associations.

8. Film comestible suivant la revendication 1, dans lequel la charge est une charge en masse.

9. Film comestible suivant la revendication 1 ou 8, dans lequel la maltodextrine a un équivalent en dextrose inférieur à 20.

10. Film comestible suivant la revendication 8, dans lequel la maltodextrine représente 20 % à 40 % en poids sec du film comestible.

11. Film comestible suivant la revendication 8, dans lequel l'hydrocolloïde représente 30 % à 40 % en poids sec du film comestible.

12. Film comestible suivant la revendication 8, dans lequel la charge en masse représente 1 % à 30 % en poids sec du film comestible.

13. Film comestible suivant la revendication 1, dans lequel la maltodextrine a un équivalent en dextrose égal ou inférieur à 10.

14. Film comestible suivant la revendication 1 ou la revendication 8, dans lequel la maltodextrine a un équivalent en dextrose égal ou inférieur à 1.

15. Procédé pour la production d'un film comestible isolé pour muco-adhérence buccale, comprenant les étapes consistant :
à préparer une solution de base comprenant au moins trois types d'agents filmogènes autres que le pullulane, lesdits agents filmogènes comprenant une maltodextrine, un hydrocolloïde et une charge ; et
à traiter la solution de base pour former le film comestible, dans lequel la maltodextrine représente 5 % à 60 % en poids sec du film comestible.

16. Procédé suivant la revendication 15, dans lequel l'hydrocolloïde représente 10 % à 50 % en poids sec du film comestible et la charge représente 1 % à 30 % en poids sec du film comestible.

17. Procédé suivant la revendication 15, dans lequel la maltodextrine a un équivalent en dextrose inférieur à 20.

18. Procédé suivant la revendication 15, dans lequel la solution de base est traitée en ajoutant une quantité thérapeutiquement efficace d'un médicament choisi entre un agent d'ajustement du pH, un agent pour les soins buccaux, un agent rafraîchisseur d'haleine, un agent pharmaceutique, un agent nutraceutique, un agent stimulant la production de salive, une vitamine, une substance minérale, un agent antimicrobien, un agent anti-plaque, un agent anti-gingivite, un agent destiné à lutter contre le tartre ou les caries et leurs associations.

19. Film comestible suivant l'une quelconque des revendications 1 à 14, comprenant en outre un médicament destiné à être utilisé dans une méthode de traitement buccal dans une cavité buccale.

20. Utilisation du film comestible suivant la revendication 19, dans la production d'un médicament destiné à un traitement buccal dans une cavité buccale.

21. Utilisation suivant la revendication 20, dans laquelle l'hydrocolloïde représente 10 % à 50 % en poids sec du film comestible et une charge représente 1 % à 30 % en poids sec du film comestible.

22. Utilisation suivant la revendication 20, dans laquelle la maltodextrine a un équivalent en dextrose inférieur à 20.

23. Utilisation suivant la revendication 20, dans laquelle le médicament est choisi entre un agent d'ajustement du pH, un agent pour les soins buccaux, un agent rafraîchisseur d'haleine, un agent pharmaceutique, un agent nutraceutique, un agent stimulant la production de salive, une vitamine, une substance minérale, un agent antimicrobien, un agent anti-plaque, un agent anti-gingivite, un agent destiné à lutter contre le tartre ou les caries et leurs associations.

24. Utilisation suivant la revendication 20, dans laquelle le médicament est libéré dans la cavité buccale pour traiter l'haleine fétide, la plaque dentaire, la gingivite, la xérostomie, la sécheresse buccale, les odeurs désagréables buccales ou leurs associations.
